(19) Europäisches Patentamt
European Patent Office
Office européen des brevets

(11) Veröffentlichungsnummer: **0 381 026**
**A2**

(12) **EUROPÄISCHE PATENTANMELDUNG**

(21) Anmeldenummer: **90101370.6**

(22) Anmeldetag: **24.01.90**

(51) Int. Cl.5: **C07C 15/38, C07C 15/20, C07C 43/164, A61B 5/00, G01N 21/64**

(30) Priorität: **03.02.89 CH 393/89**

(43) Veröffentlichungstag der Anmeldung: **08.08.90 Patentblatt 90/32**

(84) Benannte Vertragsstaaten: **BE CH DE FR GB IT LI NL**

(71) Anmelder: **F. HOFFMANN-LA ROCHE AG**
**Postfach 3255**
**CH-4002 Basel(CH)**

(72) Erfinder: **Barner, Richard, Dr.**
**Traubenweg 16**
**CH-4108 Witterswil(CH)**
Erfinder: **Huber, Walter, Dr.**
**Ziegelhofweg 62**
**CH-4303 Kaiseraugst(CH)**
Erfinder: **Hübscher, Josef**
**Säspelstrasse 1**
**CH-4208 Nunningen(CH)**
Erfinder: **Müller, Francis**
**Seltisbergerstrasse 18**
**CH-4059 Basel(CH)**

(74) Vertreter: **Kloter, Rudolf et al**
**Postfach 3255 Grenzacherstrasse 124**
**CH-4002 Basel(CH)**

(54) **Optischer Sauerstoffsensor.**

(57) Es wird ein optischer Sauerstoffsensor beschrieben, der auf einer Uebertragung elektromagnetischer Energie von einem Donor auf einen Akzeptor basiert. Als Donor wird ein Pyrenderivat verwendet und als Akzeptor wird ein Perylenderivat verwendet.

**EP 0 381 026 A2**

## Optischer Sauerstoffsensor

Die vorliegende Erfindung betrifft einen optischen Sauerstoffsensor. Derartige Sensoren bestehen vielfach aus einem lichtleitenden Medium, auf das eine sauerstoffsensitive Schicht aufgebracht wurde. Solche Sensoren können einem Patienten implantiert werden und dienen insbesondere zur Überwachung des Sauerstoffgehaltes bei Intensivpatienten.

Optische Sauerstoff($O_2$)sensoren sind in der Patentliteratur und der Literatur beschrieben, vgl. z.B. Europäische Patentanmeldung, Veröffentlichungs-Nr. 91,390, Europäische Patentanmeldung, Veröffentlichungs-Nr. 252,578 sowie Journal of Optical Sensors, 1986, Vol. 1, No. 1, pp. 43 -67.

Zum Beispiel kann bei diesen Sensoren Licht über eine Optik und einen dichroitischen Spiegel in eine Glasfaser (lichtleitendes Medium) eingekoppelt werden. Dies regt am Ende der Glasfaser in der $O_2$-sensitiven Schicht den $O_2$-Indikator an. Das Fluoreszenzlicht des $O_2$-Indikators wird in der Glasfaser gesammelt und durch diese wieder an den Eingang der Faser zurückgeführt. Dieses Fluoreszenzlicht wird über den dichroitischen Spiegel, Interferenzfilter und Linsen dem Photomultiplier zugeführt. Aus dem Signal des Photomultipliers werden unter zuhilfenahme einer Auswerteelektronik Aussagen über Fluoreszenzintensität bzw. Fluoreszenzlebensdauer möglich. Die beiden Grössen können in Bezug gesetzt werden zu der $O_2$-Konzentration in der Nähe der $O_2$-sensitiven Schicht.

Das $O_2$-sensitive Element eines $pO_2$-Sensors kann auf zwei grundsätzlich verschiedene Arten ausgebildet sein.

Die $O_2$-sensitive Schicht kann auf das Faserende aufgebracht werden. Das Anregungslicht tritt aus dem Faserende aus und regt die Fluoreszenz der $O_2$-Indikatoren an. Da das abgestrahlte Fluoreszenzlicht isotrop verteilt ist, wird ein Teil wieder in die Faser eingekoppelt und durch die Faser dem Photomultiplier zugeführt.

Die sensitive Schicht kann über eine gewisse Strecke die Auskleidung der Faser ersetzen. Das Anregungslicht wird in dieser Ausbildung des sensitiven Elementes über den gesamten Bereich der sensitiven Schicht im Kern der Glasfiber geführt. Die Wechselwirkung der $O_2$-Indikatoren mit dem Anregungslicht erfolgt über das sogenannte "evanescente" Feld (Teil des elektromagnetischen Feldes, welches bei Totalreflektion in das optisch dünnere Medium eindringt). Von dieser Anregung sind nur Indikatoren betroffen, welche sich innerhalb der Reichweite diese evanescenten Feldes befinden. Die Einkopplung des Fluoreszenzlichtes erfolgt hier über das "evanescente" Feld. Die $O_2$-sensitive Schicht besteht aus $O_2$-Indikatormolekülen. Die $O_2$-Indikatormoleküle werden in Polymeren gelöst, welche unter dem Sammelbegriff Silikone zusammengefasst sind. Der einfachste Vertreter dieser Klasse ist Polydimethylsiloxan. Eine Vielzahl von Gruppen (z.B. Alkyl, Phenyl, Trifluoropropyl, Vinyl, Wasserstoff etc.) kann Methyl als Substituent an Silizium ersetzen. Darüberhinaus können einzelne repetierende Einheiten durch Verzweigungspunkte ersetzt sein. Diese erlauben eine Quervernetzung einzelner linearer Ketten. Die Folgen dieser Quervernetzung sind hochmoleku lare Silikongummis. Silikon wird aufgrund seiner hohen $O_2$-Permeabilität bzw. $O_2$-Löslichkeit des Trägermatierals für die $O_2$-Indikatoren gewählt.

Die Darstellung der Silikonmatrix erfolgt z.B. ausgehend von sogenannten Prepolymeren, welche z.B. unter dem Einfluss von Luftfeuchtigkeit aushärten. Hierzu wird das Prepolymer in einem Lösungsmittel gelöst, welches die $O_2$-Indikatormoleküle enthält. Diese Lösung wird in einer dünnen Schicht auf das Glassubstrat aufgebracht. In Kontakt mit Umgebungsfeuchtigkeit erfolgt das Aushärten des Polymers. Als Alternative dazu kann die Polymerisation auch in Abwesenheit der $O_2$-Indikatoren erfolgen. Unter Verwendung eines Lösungsmittels, in welchem das Polysilikon aufquillt, können diese anschliessend eindifundiert werden.

Erfindungsgemäss besteht die $O_2$-sensitive Schicht nicht aus einer einzigen Indikatorverbindung, sondern aus einem Donor-Akzeptor-Paar, wie es z.B. in Applied Spectroscopy, Vol. 42, No. 6, 1988 (1009 - 1011) beschrieben ist. Die dort beschriebene Kombination von Pyren und Perylen bringt die idealen spektralen Anforderungen mit, die an ein Donor-Akzeptor-Paar für einen $O_2$-Nachweis gestellt werden. Der angeregte Zustand des Pyrens wird sehr effizient durch Sauerstoff gequencht. Das Emissionsspektrum des Pyrens (Donor) überlappt gut mit dem Absorptionsspektrum des Perylens. Perylen selber besitzt ein Emissionsmaximum bei = 440 und 470 nm. Anregungswellenlänge für den Donor (Pyren) und Emissionswellenlänge des Akzeptors (Perylen) sind also um mehr als 100 nm voneinander separiert.

Der einzige Nachteil dieses Donor-Akzeptor-Paares liegt in der geringen Löslichkeit von Pyren und Perylen in Silikon ($<10^{-3}$ mol/dm$^3$). Die entscheidende Grösse für einen effizienten Energietransfer ist der Abstand zwischen Donor und Akzeptor. Der geeignete Abstand kann über die Konzen tration eingestellt werden, wenn die entsprechenden Moleküle genügend ( $10^{-2}$ mol/dm$^3$) löslich sind. Hohe Konzentration an $pO_2$-Indikator ist auch notwendig für das Signal-Rausch-Verhältnis, Miniaturisierung der sensitiven Schicht

und Langlebigkeit des Sensors.

Im Rahmen der vorliegenden Erfindung wurde nun gefunden, dass durch eine geeignete Substitution des Pyrens und des Perylens Donor-Akzeptor-Paare erhalten werden, welche einerseits besser löslich sind als Pyren und Perylen in Silikon und die andererseits die $O_2$-Indikatoreigenschaften beibehalten.

Die vorliegende Erfindung betrifft somit einen optischen Sauerstoffsensor basierend auf einer Übertragung elektromagnetischer Energie von einem Donor auf einen Akzeptor, welcher dadurch gekennzeichnet ist, dass der Donor ein Pyrenderivat der allgemeinen Formel

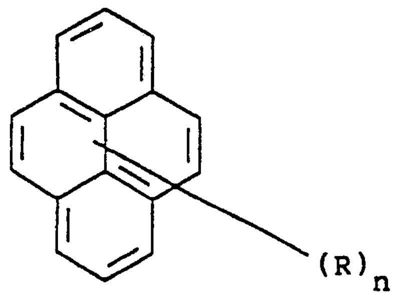

I

ist,
wobei R eine Alkylgruppe mit bis 30 C-Atomen, $-CH_2-OR$, $-R'-O-R''$ oder -OR bedeutet, wobei n eine ganze Zahl von 1 - 10 bedeutet, R′ eine Alkylengruppe von 1 - 29 C-Atomen und R″ ein Alkylgruppe von 1 - 29 C-Atomen ist, wobei jedoch R′ und R″ höchstens 30 C-Atome aufweisen,
und dass der Akzeptor ein Perylenderivat der allgemeinen Formel

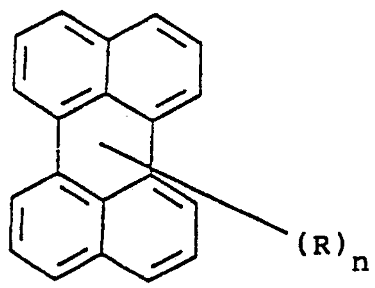

II

ist, wobei R und n die genannte Bedeutung haben.

Als Donoren kommen insbesondere Dioctadecylpyren, Tetraoctadecylpyren, 1-(Methoximethyl)pyren sowie 1-(Octadecyloximethyl)pyren in Betracht. Als Akzeptoren eignen sich insbesondere Dioctadecylperylen, Tetraoctadecylperylen sowie 3-(Octadecyloximethyl)perylen. Mit Ausnahme von 1-(Methoximethyl)-pyren sind diese Verbindungen neu und bilden ebenfalls Bestandteil der vorliegenden Erfindung.

Die Verbindungen der Formeln I oder II, worin R eine Alkylgruppe mit bis zu 30 C-Atomen darstellt, können in an sich bekannter Weise durch Alkylierung von Pyren oder Perylen mit dem entsprechenden Alkylhalogenid in einer Friedel-Crafts Reaktion mit Aluminiumchlorid als Katalysator in einem inerten Lösungsmittel wie Tetrachlorkohlenstoff, Methylenchlorid oder in desaktivierten flüssigen Aromaten, wie Nitrobenzol, Chlorbenzol, vorzugsweise Methylenchlorid bei 0 - 30°, bevorzugt Raumtemperatur hergestellt werden. Die Einführung der Alkylgruppen erfolgt entsprechend dem für den betreffenden Aromaten charakteristische Substitutionsmuster der elektrophilen Substition. Nach üblicher Aufarbeitung mit Eiswasser/Hexan können die Alkylierungsprodukte durch Chromatographie in die Di-Tetra- und höheralkylierten Verbindungen getrennt werden.

Die Verbindungen der Formeln I oder II, worin R $-CH_2-OR$, $-R'-O-R''$- oder OR bedeuten, können durch Überführung der entsprechenden Hydroxyverbindung in das Alkoholat durch Deprotonierung mit Natriumhybrid bei 0° bis Raumtemperatur in einem inerten, für die anschliessende Alkylierung günstigen Lösungsmittel, wie Tetrahydrofuran oder N,N-Dialkylformamid und Umsetzung (nach abgeklungener $H_2$-Entwicklung) mit dem entsprechenden Alkylhalogenid bei Raumtemperatur zur Alkoxyverbindung, hergestellt werden.

Die nachfolgenden Beispiele erläutern die Erfindung:

Beispiel 1

Es wurden 2,5 g (10mMol) Perylen als Lösung in 10 ml trokkenem Methylenchlorid zugetropft zu einer Suspension von 3,5 g (10,5mMol) 1-Bromoctadecan und 1 g (8mMol) Aluminiumchlorid in 10 ml Methylenchlorid und während 5 Std. bei Raumtemperatur gerührt. Es wurde mit Eiswasser hydrolisiert, mit Hexan extrahiert und eingeengt. Das Rohprodukt wurde an Kieselgel mit Hexan chromatographiert. Es wurden dabei 1,2 g (15,9 %) Dioctadecylperylen und 2,1 g (16,6 %) Dioctadecylperylen (als Isomerengemisch neben Edukt und anderen Alkylierungsprodukten) als gelbe Oele erhalten. DC[Kieselgel/Hexan)RF(Edukt)-= 0,3Rf(Dioctadecylperylen/Tetraoctadecylperylen) = 0,6/0,7
Mol($C_{20}H_{12}$) = 252,32
Mol($C_{56}H_{84}$) = 757,29
Mol($C_{92}H_{156}$) = 1262,27.

Beispiel 2

Es wurden 20 g (100mMol) Pyren als Lösung in 100 ml trokkenem Methylenchlorid zugetropft zu einer Suspension von 35 g (105mMol) 1-Bromoctadecan und 5 g (40mMol) Aluminiumchlorid in 10 ml Methylenchlorid und während 5 Std. bei Raumtemperatur gerührt. Es wurde mit Eiswasser hydrolisiert, mit Hexan extrahiert und eingeengt. Das Rohprodukt wurde an Kieselgel mit Hexan chromatographiert. Es wurden dabei 16 g (22,6 %) Dioctadecylpyren und 19 g (15,7 %) Dioctadecylpyren (als Isomerengemisch neben Edukt und anderen Alkylierungsprodukten) als gelbe Oele erhalten. Die Produkte wurden im IR, NMR und MS und Mikroanalyse charakterisiert. DC(Kieselgel/Hexan)Rf(Edukt) = 0,4Rf(Diocta decylpyren/ Tetraoctadecylpyren) = 0,7/0,8
Mol($C_{16}H_{10}$) = 202,26
Mol($C_{52}H_{82}$) = 707,23
Mol($C_{88}H_{154}$) = 1212,21.

Beispiel 3

Es wurden 232 mg (1mMol) 1-Hydroximethylpyren in 2 ml trockenem THF gelöst und mit 30 mg (1,2mMol) Natriumhydrid (80 % in Mineralöl) versetzt und 30 Min. bei Raumtemperatur gerührt. Dann wurde 1 ml (ca. 1mMol) Methyliodid zugetropft und 3 Std. bei Raumtemperatur gerührt. Es wurde mit Eiswasser versetzt, mit Toluol extrahiert und die organische Phase durch Kieselgel filtriert und eingeengt. Es wurden 243 mg (99 %) 1-(Methoximethyl)pyren als bräunliches Oel erhalten und im MS charakterisiert. DC-(Kieselgel/Toluol)Rf(Edukt) = 0,1Rf(Produkt) = 0,5.

Beispiel 4

Eine Lösung von 186 mg (0,8mMol) 1-(Hydroximethyl)pyren in trockenem N,N-Dimethylformamid (DMF) wurde bei 0° C mit 100 mg einer 55 - 60%-igen Suspension von Natriumhydrid in Mineralöl (ca. 2mMol Natriumhydrid) unter Rühren versetzt und nach abgeklungener Wasserstoffentwicklung noch 30 Min. bei Raumtemperatur gerührt. Zu der erhaltenen Lösung hat man eine Lösung von 270 mg (0,8mMol) Octadecylbromid in 1 ml trockenem DMF unter Rühren zugetropft und anschliessend das Reaktionsgemisch noch über Nacht gerührt (Raumtemperatur). Durch Filtration über "Hyflo" wurden die Salze abgetrennt und das Filtrat direkt an Kieselgel chromatographiert, wobei 363 mg (85%) 1-(Octadecyloximethyl)pyren kristallin erhalten wurden; Smp = 67-69°.
Die Verbindung wurde im Massenspektrum charakterisiert (M+ = 484)
DC(Kieselgel/Toluol)Rf = 0,8Rf(Hydroximethylpyren) = 0,1
Mol($C_{35}H_{48}O$) = 484,73.

Beispiel 5

In Analogie zu Beispiel 4 wurden 115 mg (0,42mMol) 3-(Hydroximethyl)-perylen mit Octadecylbromid zum 3-(Octadecyloxymethyl)perylen umgesetzt (135mg; 60%)
Smp = 101-103°
MS:M + = 534
DC(Kieselgel/Toluol)Rf = 0,9
Mol(C30H50O) = 534,79.

Beispiel 6

(Herstellung einer sauerstoffsensitiven Schicht)

1 g Elastosil E43 (Wacker Chemie) wird in einer Toluollösung (1 ml) des Energiedonors Dioctadecylpyren (0,001 m) und des Energieakzeptors Dioctadecylperylen (0,001 m) gelöst. Die resultierende dickflüssige Masse wird in gewünschter Schichtdicke auf das Substrat aufgebracht. Das Aushärten des Polymers erfolgt über 24 h.

Die Figur 1:a) und Figur 1:b) zeigen schematisch zwei optische Sauerstoffsensoren mit einer $O_2$-sensitiven Schicht gemäss Beispiel 6.

**Ansprüche**

1. Optischer Sauerstoffsensor basierend auf einer Uebertragung elektromagnetischer Energie von einem Donor auf einen Akzeptor, dadurch gekennzeichnet, dass der Donor ein Pyrenderivat der allgemeinen Formel

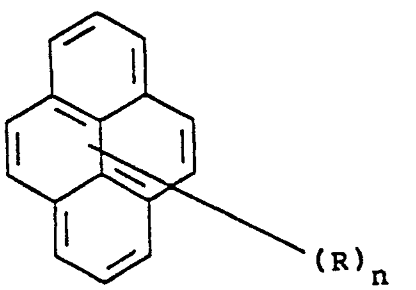

I

ist,
wobei R eine Alkylgruppe mit bis 30 C-Atomen, $-CH_2-OR$, $-R'-O-R''$ oder -OR bedeutet, wobei n eine ganze Zahl von 1 - 10 bedeutet, R′ eine Alkylengruppe von 1 - 29 C-Atomen und R″ eine Alkylgruppe von 1 - 29 C-Atomen ist, wobei jedoch R′ und R″ höchstens 30 C-Atome aufweisen,
und dass der Akzeptor ein Perylenderivat der allgemeinen Formel

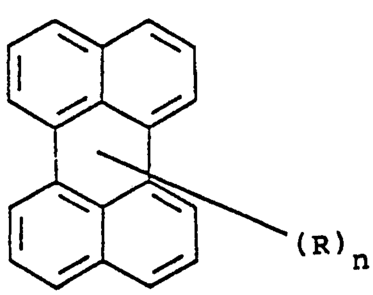

II

ist, wobei R und n die genannte Bedeutung haben.

2. Optischer Sauerstoffsensor, dadurch gekennzeichnet, dass der Donor Dioctadecylpyren ist.

3. Optischer Sauerstoffsensor, dadurch gekennzeichnet, dass der Donor Tetraoctadecylpyren ist.

4. Optischer Sauerstoffsensor, dadurch gekennzeichnet, dass der Donor 1-(Methoximethyl)pyren ist.

5. Optischer Sauerstoffsensor, dadurch gekennzeichnet, dass der Donor 1-(Octadecyloximethyl)pyren ist.

6. Optischer Sauerstoffsensor, dadurch gekennzeichnet, dass der Akzeptor Dioctadecylperylen ist.

7. Optischer Sauerstoffsensor, dadurch gekennzeichnet, dass der Akzeptor Tetraoctadecylperylen ist.

8. Optischer Sauerstoffsensor, dadurch gekennzeichnet, dass der Akzeptor 3-(Octadecyloximethyl)-perylen ist.

9. Pyren- und Perylenderivate, nämlich Dioctadecylpyren, Tetraoctadecylpyren, 1-(Octadecyloximethyl)-pyren, Dioctadecylperylen, Tetraoctadecylperylen, sowie 3-(Octadecyloximethyl)perylen

Figur 1 : a) Sauerstoffsensor mit der sauerstoffsensitiven Schicht stirnseitig auf das lichtleitende Substrat aufgebracht (Direkte Anregung der Fluoreszenz).

b) Sauerstoffsensor mit sauerstoffsensitiver Schicht als Cover auf der lichtleitenden Schicht (Anregung der Fluoreszenz über das evanescente Feld).

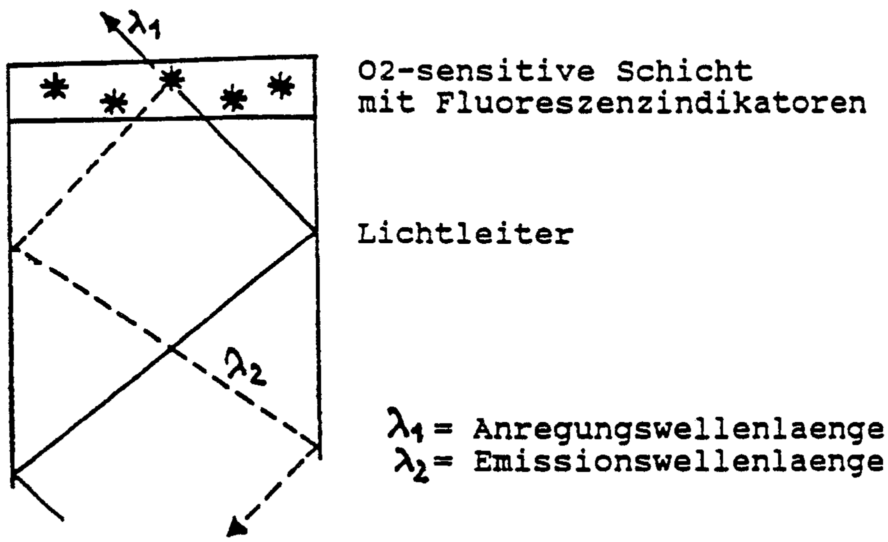


Figur 1:a)

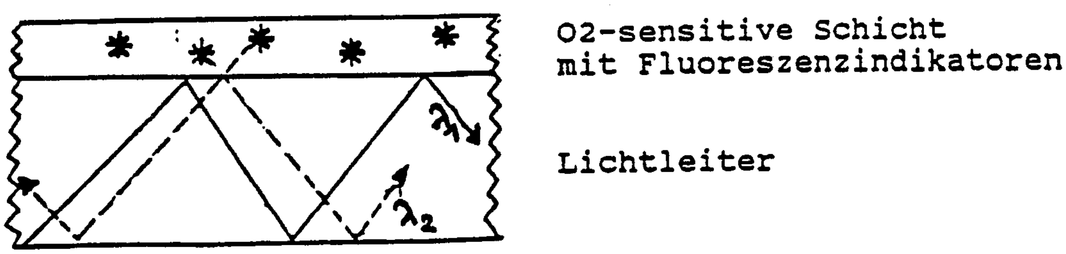


Figur 1:b)